Europäisches Patentamt

European Patent Office

Office européen des brevets

(11) Veröffentlichungsnummer: **0 618 298 A2**

# EUROPÄISCHE PATENTANMELDUNG

(21) Anmeldenummer: 94103883.8

(22) Anmeldetag: **14.03.94**

(51) Int. Cl.5: **C12N 15/80**, C12N 1/15, C12P 21/00

(30) Priorität: **20.03.93 DE 4309071**

(43) Veröffentlichungstag der Anmeldung:
**05.10.94 Patentblatt 94/40**

(84) Benannte Vertragsstaaten:
**AT BE CH DE DK ES FR GB IT LI NL PT SE**

(71) Anmelder: **HOECHST AKTIENGESELLSCHAFT**
**Brüningstrasse 50**
**D-65929 Frankfurt am Main (DE)**

(72) Erfinder: **Kück, Ulrich, Dr.**
**Zum Felde 11**
**D-44797 Bochum (DE)**
Erfinder: **Menne, Stephan**
**Cramerstrasse 40**
**D-44793 Bochum 1 (DE)**
Erfinder: **Walz, Markus, Dr.**
**Leifacker 1**
**D-44892 Bochum (DE)**

(54) Bidirekfionaler Promotor des pcbAB- und pcbC- Gens von Acremonium chrysogenum und seine Verwendung.

(57) Die Erfindung betrifft den bidirektionalen Promoter des pcbAB- und pcbC-Gens von Acremonium chrysogenum und seine Verwendung für die Herstellung von Fremdproteinen und für die Bestimmung der Genexpression von Reportergenen in Acremonium chrysogenum.

Die Erfindung betrifft den bidirektionalen Promoter des pcbAB- und pcbC-Gens von Acremonium chrysogenum und seine Verwendung für die Herstellung von Fremdproteinen und für die Bestimmung der Genexpression von Reportergenen in Acremonium chrysogenum.

Bei Pilzen wird die Genexpression vornehmlich auf der Transkriptionsebene reguliert. Die Regulierung der Transkription ist im allgemeinen abhängig von DNA-Sequenzmotiven im 5'- und 3'-nichtkodierenden Bereich eines Gens ("cis-wirksame Elemente"), DNA-Sequenzen innerhalb der kodierenden Region ("cis-wirksame Elemente") und Proteinen, die sequenz-spezifisch über DNA-bindende Domänen mit bestimmten DNA-Sequenzmotiven interagieren können (sog. "trans-wirksame Elemente").

Interessanterweise weisen bei Hyphenpilzen die Promotorelemente häufig eine variable DNA-Sequenz auf. So kann beispielsweise die sogenannte TATA-Box bei Hyphenpilzen, wie z. B. im NADP-spezifischen Glutamatdehydrogenasegen von Neurospora crassa die Konsensussequenz TATAAA besitzen. Sie kann aber auch als AT reiche Sequenz ausgebildet sein, wie im aldA-Gen von Aspergillus nidulans (A. nidulans). Oder aber es existiert keine Sequenz, die eine Homologie zur TATA-Konsensussequenz aufweist, wie im trpC-Gen von A. nidulans.

Die CAAT-Box wurde bisher nur in wenigen pilzlichen Genen gefunden wurde, so z. B. im pgk-Gen von A. nidulans. Dagegen befindet sie sich z. B. nicht im argB-Gen oder im trpC-Gen von A. nidulans.

Zusätzlich wurden Sequenzmotive gefunden, die über größere Entfernungen spezifisch für die Aktivität des jeweiligen Promotors sind (z. B. "Enhancer", UAS (upstream activation site), "Silencer") und mit regulativen Proteinen interagieren. Konservierte DNA-Sequenzen innerhalb und außerhalb der transkribierten Region sind oft direkte bzw. umgekehrte ("inverted") kurze Sequenzwiederholungen, die ebenfalls als Bindestellen von regulativ wirkenden Proteinen fungieren. So wurde z. B. nachgewiesen, daß nach einem Temperaturstreß Hitzeschock-Transkriptionsfaktoren an derartigen Sequenzen in sog. Hitzeschockgenen binden. Proteine, die sequenz-spezifisch mit bestimmten DNA-Bereichen interagieren können, sind neben der DNA-abhängigen RNA Polymerase II sog. Transkriptionsfaktoren. Diese sind an der Regulation der Transkription beteiligt. Die Expression der meisten Gene von Hyphenpilzen wird durch regulativ wirkende Genprodukte kontrolliert.

Gutiérrez et al. (1991) fanden nun, daß das pcbAB-Gen mit dem pcbC-Gen von Cephalosporium acremorium über einen intergenischen Bereich verbunden ist.

Die Aufgabe der vorliegenden Erfindung war daher, diesen intergenischen Bereich zu sequenzieren und zu charakterisieren. Bei der Lösung dieser Aufgabe wurde nun überraschenderweise gefunden, daß dieser intergenische Bereich einen bidirektionalen Promoter enthält, d. h. einen Promoter, der in die 5'- wie auch in die 3'-Richtung aktiv ist. Dabei steuert er gleichzeitig die Expression von zwei Genen, die an der 5'-Seite bzw. 3'-Seite mit dem Promoter verbunden sind. Auch war es überraschend, daß die Promoteraktivität in beiden Richtungen unterschiedlich stark ist.

Ein Gegenstand der Erfindung ist daher ein DNA-Segment gemäß Tab. 2 oder Teile davon, die eine bidirektionale Promotoraktivität besitzen und ein DNA-Segment, das mit einer DNA gemäß Tab. 2 unter stringenten Bedingungen, insbesondere bei 68° C in 6 x SSC-Puffer (NaCl-Citratpuffer), hybridisiert und eine bidirektionale Promotoraktivität besitzt.

Die Herstellung des erfindungsgemäßen Promotors erfolgt anhand der Sequenz der Tabelle 2 entweder chemisch oder gentechnisch nach dem Fachmann bekannten Methoden.

Der erfindungsgemäße Promotor kann für die Regulation der Genexpression von Fremdproteinen in Acremonium chrysogenum verwendet werden. Zusätzlich kann der Promotor für die Expression von Fremdproteinen in Aspergillus niger verwendet werden, da U. Kück et al. (1989) bereits fanden, daß die 5'-Region des pcbC-Gens auch in Aspergillus niger aktiv ist.

Ein weiterer Gegenstand der Erfindung ist daher ein Verfahren zur Herstellung eines gewünschten Proteins in einer Wirtszelle, insbesondere Acremonium chrysogenum oder Aspergillus niger, wobei der erfindungsgemäße Promotor funktionell mit ein oder zwei proteinkodierenden DNA-Segmenten verbunden wird, so daß der Promotor die Expression des oder der gewünschten Proteine in der Wirtszelle steuert. Daher gehören auch zum Gegenstand der Erfindung Vektoren bzw. Expressionsvektoren, die den Promotor alleine zum Zwecke der Umklonierung oder in Kombination mit proteinkodierenden DNA-Segmenten enthalten und ferner die mit diesen Vektoren transformierten Wirtszellen, insbesondere Acremonium chrysogenum und Aspergillus nidulans. Die Kultivierung der Wirtszellen und die Isolierung des gewünschten Proteins erfolgt nach allgemein bekannten Verfahren.

Besonders bevorzugt ist die Genexpression in Acremonium chrysogenum unter der Kontrolle des bidirektionalen Promotors. Die dazu verwendeten Vektoren besitzen im allgemeinen die folgenden Bestandteile:

- ein selektionierbares Markergen;

- ein sog. Reportergen, dessen Expression quantitativ im transformierten Organismus analysiert werden soll (dies kann auch der Selektionsmarker sein);
- der erfindungsgemäße Promotor, der an den 5'-Bereich eines Gens, beispielsweise eines Reportergens, in Form einer Transkriptions- oder Translations-Fusion kloniert ist und somit die Expression des Gens steuert;
- einen Terminator bzw. jede andere DNA-Sequenz, die an den 3'-Bereich des Gens fusioniert ist und die Transkriptions-Termination bestimmt.

Unter einem Reportergen versteht man solche Gene, deren Produkte leicht nachweisbar und quantifizierbar sind. Die Quantifizierbarkeit erfolgt in der Regel durch enzymatische Teste, die z. B. ein einfaches und schnelles visuelles Nachweisverfahren ermöglichen. Dieses Nachweisverfahren kann in der Regel zur qualitativen und auch quantitativen Messung eingesetzt werden.

In der folgenden Tabelle werden bei Pilzen häufig verwendete Reportergene aufgeführt.

Tab. 1

| Häufig verwendete Reportergene bei Pilzen, deren Genprodukte (Enzyme) leicht nachweisbare Reaktionen katalysieren (nach Herrera-Estrella et al. 1988; Richard et al. 1992): | | |
|---|---|---|
| Gen | Genprodukt | Rezipient |
| lacZ | $\beta$-D-Galaktosidase | Saccharomyces cerevisiae Aspergillus nidulans Penicillium chrysogenum |
| gusA (syn.: uidA) | $\beta$-D-Glukuronidase | Saccharomyces cerevisiae Schizosaccaromyces pombe Aspergillus nidulans Aspergillus niger Acremonium typhinum Ustilgo maydis Fulvia fulva |
| cat | Chloramphenicol-Acetyltransferase | Saccharomyces cerevisiae |

Ein weiterer Gegenstand der Erfindung ist daher auch die Verwendung des erfindungsgemäßen Promotors zur quantitativen Bestimmung der Genexpression von Reportergenen in Acremonium chrysogenum.

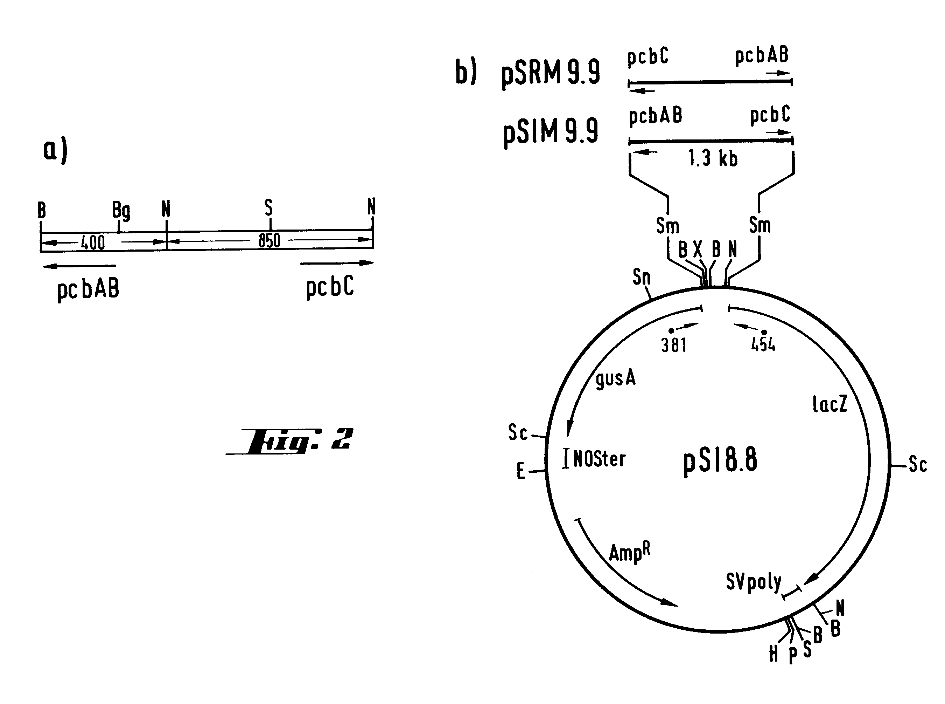

Schließlich kann der in Fig. 2 beschriebene Vektor pSI8.8 oder äquivalente Varianten davon mit anderen Marker- oder Reportergenen oder Terminatorsequenzen auch zum Auffinden weiterer Promotoren aus Acremonium chrysogenum verwendet werden.

Beschreibung der Figuren:

Fig. 1: Physikalisch-genetische Karte der pcbAB-pcbC-Genregion. Der näher gekennzeichnete Bereich wurde bei der DNA-Sequenzierung analysiert. Zu diesem Zweck fand eine Subklonierung von Sau3A-Restriktionsfragmenten in den Vektor pUC19/BamHI statt.

Fig. 2: Konstruktion der Transformations-Vektoren

a) Physikalisch-genetische Karte der - beiden Cephalosporin C-Genen gemeinsam - 1.2 kb großen DNA-Region. Die Kartierung der Restriktions-Schnittstellen erfolgte für die Enzyme BamHI, BglII, NcoI und SalI. Das aus dem Vektor pIPNSB1 erhaltene BamHI/NcoI-Fragment enthält die 5'-nichtkodierenden Sequenzen des pcbAB- und des pcbC-Gens. Die Pfeile symbolisieren die entgegengesetzt orientierten Transkriptionsrichtungen der beiden Promotoren. Die Größe des BamHI/NcoI- und des NcoI/NcoI-Fragments ist in bp angegeben.

b) Physikalisch-genetische Karten der Transformations-Vektoren pSI8.8, pSIM9.9 und pSRM9.9. Die Transkriptions-Richtungen der Reportergene lacZ und gusA sowie des Ampicillin-Resistenzgens sind durch lange Pfeile symbolisiert. Die kurzen Pfeile geben die Position und die Sequenzierungsrichtung der bei der Sequenzierung als Primer verwendeten Oligonucleotide an.

Abkürzungen: B, BamHI; Bg, BglII; E, EcoRI; H, HindIII; N, NotI; Nc, NcoI; P, PstI; S, SalI; Sc, SacI; Sm, SmaI; Sn, SnaBI; X, XbaI; Amp$^R$, Ampicillin-Resistenzgen; gusA, Gen, kodiert die $\beta$-D-Glukuronidase; lacZ, Gen, kodiert die $\beta$-D-Galaktosidase; NOSter, Nopalin-Synthase-Polyadenylierungs-Signal; pcbC, Gen, kodiert die Isopenicillin-N-Synthetase; SVpoly, SV40-Polyadenylierungs-Signal; Oligonukleotid Nr. 381 (komplementär dem gusA-Gen); Oligonukleotid Nr. 454 (komplementär dem lacZ-Gen).

Zum Offenbarungsgehalt der vorliegenden Anmeldung soll auch der Inhalt der deutschen prioritätsbegründenden Anmeldung mit der Nummer P 4309071.0 zählen.

Die folgenden Beispiele sollen nun die Erfindung näher erläutern.

Beispiele:

1. Isolierung und DNA-Sequenzierung des pcbAB-pcbC-intergenischen Bereiches.

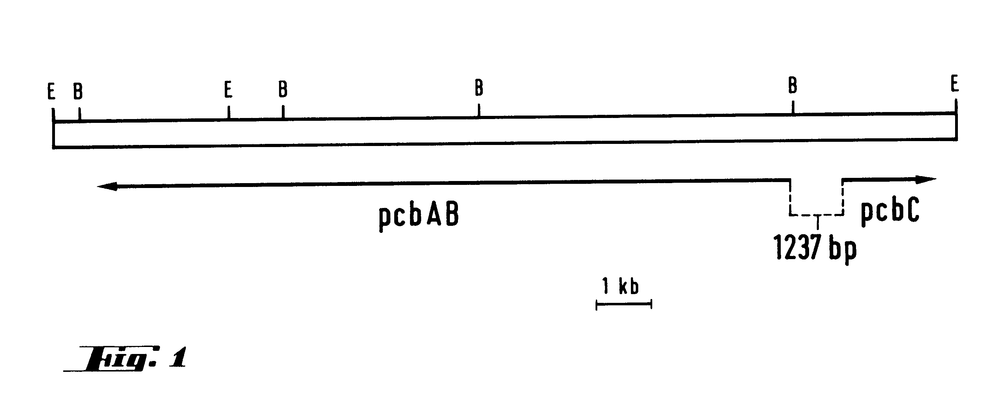

In der Fig. 1 ist die Gen-Anordnung des pcbAB- und des pcbC-Gens von Acremonium chrysogenum wiedergegeben. Die Pfeile geben die Transkriptionsrichtung der Gene wieder. Dadurch wird deutlich, daß beide Gene von unterschiedlichen DNA-Strängen kodiert werden. Der Bereich zwischen den beiden Startkodonen ist in der Fig. 1 näher markiert worden und konnte durch Subklonierung für die DNA-Sequenzierung vorbereitet werden. Die DNA-Sequenzierung erfolgte nach den bekannten Methoden der Molekularbiologie unter Verwendung von sogenannten Primern, die individuell von uns synthetisiert wurden und im folgenden Abschnitt näher benannt sind. Die ermittelte DNA-Sequenz ist in der Tab. 1 als Einzelstrang wiedergegeben. Terminiert wird die Sequenz von den Start-Kodonen für das pcbAB- bzw. für das pcbC-Gen. Beide Gene werden von unterschiedlichen DNA-Strängen kodiert und besitzen deshalb eine invers zueinander angeordnete Richtung der Transkription. Die beiden Startkodonen flankieren eine Sequenz mit einer Länge von 1231 Nukleotiden, die nicht im Verlauf der Genexpression translatiert wird. Das Gen stammt von Acremonium chrysogenum-Stamm H780 (Kück et al., 1989).

Methode: DNA-Sequenzierung

Plasmid-DNA wurde nach der Didesoxy-Kettenabbruch-Methode (Sanger et al. 1977) unter Verwendung des T7 Polymerase™-Sequenzierungskits (Pharmacia Fine Chemicals AB, Uppsala, Schweden) sequenziert. Die Sequenzierung mit (alpha-$^{35}$S) dATP erfolgte nach Vorschrift des Herstellers. Die Proben wurden in Puffer-Gradientengelen nach Biggin et al. (1983) elektrophoretisch aufgetrennt. Zur DNA-Sequenzierung wurden als Startermoleküle (Synonym Primer) neben den kommerziell erwerblichen M13-Primern folgende von uns synthetisierte Oligonukleotide verwendet:
Oligonukleotid Nr. 316 (5'-AGGCCAATGCATGCATCC);
Oligonukleotid Nr. 448 (5'-AACAGTCTATCGAAGCTC);
Oligonukleotid Nr. 225 (5'-AAAGGGATATTCAAGTATTCG).
Die Position der entsprechenden Oligonukleotide ist in der Sequenz der Tab. 2 wiedergegeben.

```
                10        20        30        40        50        60
CACGGACCGG ATCCAGCAGT CAGGGGCTGC GGCTGCGAAG CCGTTGCGGT GGATGGCACC
pcbAB
            '70       80        90        100       110       120
TTTTGGGATA TCAGAGCTTC CATTGCGCCT GATGTGGTAA TGTTAGAGAT AGGTCCCAAC

               130       140       150       160       170       180
TGCGGCTTCC AGAGGCTGTG AAGACATACC TGTGGTATGA CGAGGCGTCG TCGGCACCTG

               190       200       210       220       230       240
TAAGGATAGT CATGTTGATA TCAAAGGTGT ATGATAGACT GGCAGTCCAC TACTGACTGA

               250       260       270       280       290       300
GTGTGACCGA GTCCAAGGAG AAATATTTCC GCCAGTTCTT TTTCGCACTC TACGGCCGAT

               310       320       330       340       350       360
ATTTCCCAAC ACGTCGCTAG ATCTCCCAGC ACGAACGTCT TATACTCCTG GGGAATCCCG

               370       380       390       400       410       420
GACCTTTGAT ACAGGCCAAT GCATGCATCC AGCTGGCCAC CCACCCAGAC CAGTGAACTC
               316

               430       440       450       460       470       480
CAAAGCCGGC ACCGAACCCA CCAGGACCGC CTTTGTCGCC ATGGATACCC TTTCTCTCCT

               490       500       510       520       530       540
CGGCCAAGCG CGATGGCCCG CGTCTTGCAG AAAGCTGGGC AGGCTTCAGA AGTGGGCCAC

               550       560       570       580       590       600
GCTGCAGGTC GGTCCCTCGT CTGCGAGTTT AAAGTGCAAC GTGAACCAAA GCCACCCCGT

               610       620       630       640       650       660
ATGTGTGGTT CCATTGGGTT GAGAAACTGG ATTGTACGGG GTACCTCTAC CCCGAGTTCC

               670       680       690       700       710       720
TCGTCGATCG GGTACATGAT ACATTGTACA CCGTAGTAGC AGTGTGTACG GCCCATCGCC

               730       740       750       760       770       780
GTGGTGGCCC GCAGCAGCGC GAGATTCCGA CGGACGCCGT CGACTACCGG TGAGCCGCTC

               790       800       810       820       830       840
GACGGGGCGT CGAGTTGCCG GGCCAATCCC TGAGCTTCGA TAGACTGTTC CGGGCCTCAT
                                            448

               850       860       870       880       890       900
GGGTGGCGGC GTCTACATGC ACATGCATGT AACGGCGTTC CTCATCGCTT GGCCCCGCCA

               910       920       930       940       950       960
TGCAGTCTTC AGGGACCAAA CTCCATCGCC GCTGCTGGAC CGTATGTAAC CCCCTCGGC

               970       980       990       1000      1010      1020
AGTGTCACCC GCAGGAGCCG GATAATCGAG ACCTTGGTCA GGCCATAAAG GCGCGTCGTG

               1030      1040      1050      1060      1070      1080
GGGAAGCTCA TATCGTATAG CAACGGGAGA CACGAGGTAG GTACTCAAGT ACACATACAC

               1090      1100      1110      1120      1130      1140
ACACCCAGCC GCCCGTATAA ACAGCTTCAA GAGGGCCGAA TACTTGAATA TCCCTTGGT
                                            225

               1150      1160      1170      1180      1190      1200
CGCTCTTCTG ATTTTCGAGG CTTCTCCTTC CGCCATCGTC ACTCACGCAT ATCTCGTCTT

               1210      1220      1230      1240      1250      1260
TCACATCTTA CACCAGGCAG GACAAACCGT CACCATG
                                   pcbC
```

Tab. 2: DNA-Sequenz der intergenischen pcbAB-pcbC-Genregion. Die Start-Kodonen (eingerahmt) der beiden Gene, die von unterschiedlichen Strängen kodiert werden, sind ca. 1,2 kb voneinander entfernt. Die Transkriptionsrichtung der Gene ist durch Pfeile gekennzeichnet. In der Sequenz wurde die Position von Oligonukleotiden eingezeichnet, die bei der DNA-Sequenzierung Verwendung fanden.

2. Konstruktion von Vektoren zur quantitativen Messung der Gen-Expression: pSI8.8, pSIM9.9, pSRM9.9

Ziel der Vektor-Konstruktionen war es, Transkriptions-Fusionen zwischen dem intergenischen Bereich der beiden Cephalosporin C-Biosynthesegenen aus A. chrysogenum (pcbAB- bzw. pcbC-Gen) mit zwei Reportergenen (lacZ- bzw. gusA-Gen) herzustellen. Mit Hilfe dieser Transkriptions-Fusionen sollte dann die gleichzeitige Expression der Reportergene unter Kontrolle des intergenischen Bereichs quantifiziert werden.

Als Ausgangsvektor wurde der von uns konstruierte Vektor pSI8.8 (Fig. 2) verwendet. Dieser Vektor enthält zwei aus E. coli stammende Reportergene mit jeweils vollständigem offenem Leserahmen (ORF) für das lacZ-Gen, welches für die $\beta$-D-Galaktosidase kodiert (MacGregor 1987) und das gusA-Gen, welches für die $\beta$-D-Glukuronidase kodiert (Jefferson et al. 1986) wurde. Die beiden Gene wurden aus Plasmiden gewonnen, die kommerziell erwerblich sind, das lacZ-Gen aus dem Vektor pSV$\beta$ (ITC Biotechnology GmbH, Heidelberg) und das gusA-Gen aus dem Plasmid pBI101 (ITC Biotechnology GmbH, Heidelberg). Aus dem Plasmid pBI101 wurde ein SalI/EcoRI-Fragment in das EcoRI/XhoI restringierte Plasmid pSVß ligiert. Das resultierende Plasmid pSI8.8 hat eine Größe von 8,8 kb. Diese beiden promoterlosen Reportergene sind auf dem Vektor pSI8.8 derart angeordnet, daß ihre Transkriptions-Richtungen entgegengesetzt zueinander orientiert sind. Um eine effiziente Termination der Reportergen-Expression sicherzustellen, sind im Vektor 3' (stromabwärts) zu diesen Genen Transkriptions-Terminatoren lokalisiert. Das gusA-Gen besitzt als Terminator eine DNA-Sequenz mit dem Nopalin-Synthase-Polyadenylierungs-Signal aus dem Ti-Plasmid von Agrobakterium tumefaciens (Bevan 1984). Das lacZ-Gen hat als Terminationssequenz das Polyadenylierungs-Signal aus dem SV 40-Virus (McGregor 1987).

Das Plasmid pIPNSB1 enthält ein 3,5 kb großes BamHI-Fragment der genomischen DNA von A. chrysogenum (Walz 1992), das in die singuläre Restriktions-Schnittstelle (Yanish-Perron et al. 1985) für das Enzym BamHI des Vektors pUC9 kloniert wurde. Das nach partieller Restriktion aus dem Vektor pIPNSB1 und unter Fig. 2a) dargestellte BamHI/NcoI-Fragment wurde nach Behandlung mit Mung-Bohnen-Nuklease in beiden möglichen Orientierungen zu den Reportergenen in den mit SmaI linearisierten Vektor pSI8.8 kloniert (Tab. 3). Die resultierenden rekombinanten Plasmide erhielten die Bezeichnungen pSRM9.9 und pSIM9.9. Beide Vektoren wurden verwendet, um die Promotorfunktion des pcbAB-pcbC-intergenischen Bereiches aufgrund der Expression zweier verschiedener Reportergene überprüfen zu können. Die Überprüfung der korrekten Transkriptions-Fusionen in den Vektoren konnte durch Restriktionsanalyse und durch Sequenzierung bestätigt werden.

## Vektor pSI8.8

```
        <---gusA
5'- TAA CAT AAGGGACTGACCA CCCGGG ............... MCS ............... CCCGGG AT
                           Sma I                                      Sma I

        lacZ---->
CGAAAGAGCCTGCTAAAGCAAAAAAGAAGTCACC ATG TCGTTT -3'
```

## Vektor pSIM9.9

```
    .   <---gusA                      <--- pcbAB                        pcbC ---->

                                      14              30            1221        1234
                                      :               :             :           :
5'- TAA CAT AAGGGACTGACCA CCC cagcagtcaggggctgc - 1190 bp ⊥ gacaaaccgtcac GGG AT

                           lacZ---->

CGAAAGAGCCTGCTAAAGCAAAAAAGAAGTCACC ATG TCGTTT -3'
```

## Vektor pSRM9.9

```
    <---gusA                         <--- pcbC                        pcbAB --->
                                     1233         1221            30            14
                                     :            :              :             :
5'- TAA CAT AAGGGACTGACCA CCC  tgacggtttgtc - 1190 bp - gcagcccctgactgctg GGG AT

                           lacZ---->

CGAAAGAGCCTGCTAAAGCAAAAAAGAAGTCACC ATG TCGTTT -3'
```

EP 0 618 298 A2

Tab. 3: DNA-Sequenzen der Übergangsbereiche in den Vektoren pSI8.8, pSIM9.9 und pSRM9.9. Die DNA-Sequenz des Ausgangsvektors pSI8.8 ist im Bereich der multiplen Klonierungsstelle (MCS) und der ATG-Startkodone der Reportergene angegeben. Kleinbuchstaben kennzeichnen DNA-Sequenzen, die von A. chrysogenum stammen. Die angegebenen Nukleotidpositionen entsprechen denen der Sequenz in der Tab. 2. Dargestellt sind die Nukleotidsequenzen im Bereich der Transkriptions-Fusionen der 5'-nichtkodierenden Bereiche des pcbAB- und des pcbC-Gens und der Reportergene lacZ und gusA in den Transformations-Vektoren pSIM9.9 und pSRM9.9.

3. Kotransformation von A. chrysogenum mit den Vektoren pSIM9.9 bzw. pSRM9.9 und dem Vektor pMW1

Die Kotransformation von A. chrysogenum erfolgte nach der Methode, die in der deutschen Patentanmeldung Nr. P 43 04 312.7 beschrieben ist. Dabei wurden protoplasierte Pilzzellen mit DNA in Anwesenheit von Polyethylenglykol und $CaCl_2$ inkubiert. Bei der Kotransformation wurden gleiche Mengen der zu transformierenden Vektor-DNA eingesetzt; pro Vektor jeweils 20 $\mu$g DNA. Für die Isolierung von A. chrysogenum-Protoplasten wurden 2 bis 2,5 Tage alte Kulturen verwendet, da bei diesem Myzelalter die höchste Ausbeute regenerierbarer Protoplasten erhalten wird. Die bei der Kotransformation von A. chrysogenum erzielten Ergebnisse zeigt die Tabelle 4.

Bei den angegebenen Werten der Tab. 4 handelt es sich um Durchschnittswerte, die in vier unabhängigen Experimenten erzielt worden sind. Die Standard-Abweichung ist angegeben. Anhand dieser Ergebnisse ist erkennbar, daß bei den verwendeten Transformations-Vektoren keine Unterschiede bezüglich der Transformationsrate und die relative Transformations-Effizienz in A. chrysogenum gleich. Die Kotransformationsrate, die aufgrund qualitative Testverfahren ermittelt wurde, beträgt im Durchschnitt 76 %. Die Transformanten wurden aufgrund ihrer Resistenz gegen Hygromycin B identifiziert. Anschließend wurde die DNA der Transformanten durch DNA-Hybridisierungsverfahren näher analysiert. Die entsprechenden Methoden sind in der deutschen Patentanmeldung P 43 04 312.7 beschrieben. Bei der DNA-Hybridisierung wurde als Sonde das gusA-Gen eingesetzt. Die Sonde enthielt somit keine bakteriellen DNA-Sequenzen. Bei 76 % aller Transformanten konnte mit der gusA-Sonde ein positives Signal im Autoradiodiagramm nachgewiesen werden. Diese Transformanten wurden in der weiteren Analyse eingesetzt.

Tab. 4: Kotransformation von A. chrysogenum mit den Vektoren pSIM9.9 bzw. pSRM9.9 und dem Vektor pMW1. Angegeben sind die Regenerationsrate (Reg.-rate, Transformationsrate (Tf.-rate; in Transformanten / $\mu$g DNA), Transformationseffizienz (relative Tf.-effizienz; in Transformanten / $\mu$g DNA und $10^8$ Protoplasten) und die Kontransformationsrate (Ko-Tf.-rate).

| Vektor | Reg.-rate | Tf.-rate | relative Tf. -effizienz | Ko-Tf.-rate |
|---|---|---|---|---|
| pMW1 | 0.25 % | 0.42 | $0.17 \times 10^{-3}$ | 77 % |
| + | ( ± 0.04) | ( ± 0.09) | ( ± $0.04 \times 10^{-3}$) | ( ± 7) |
| pSIM9.9 | | | | |
| pMW1 | 0.25 % | 0.41 | $0.18 \times 10^{-3}$ | 75 % |
| + | ( ± 0.04) | ( ± 0.09) | ( ± $0.05 \times 10^{-3}$) | ( ± 6) |
| pSRM9.9 | | | | |

4. Messung der Promotor-Aktivität des pcbAB-pcbC-intergenischen Bereiches: Quantitativer Test der $\beta$-D-Galaktosidase und $\beta$-D-Glukuronidase-Aktivität mit Hilfe von Reportergenen

Um die Regulation der Genexpession des pcbAb- und des pcbC-Gens in A. chrysogenum zu untersuchen, wurden Transkriptions-Fusionen mit zwei verschiedenen Reportergenen konstruiert. Diese Fusionen haben den Vorteil, daß die Aktivitäten der von den Reportergenen kodierten Enzyme parallel gemessen werden können. Daher kann die unter Kontrolle des pcbAB- und des pcbC-Promotors stehende Expression der Reportergene quantifiziert werden. Mit Hilfe der Vektoren pSIM9.9 und pSRM9.9 wurde eine simultane Analyse der Expressionsssignale der beiden entgegengesetzt transkribierten Cephalosporin C-Biosynthesegene durchgeführt. Insbesondere wurde untersucht, ob diese beiden Gene des sekundären Stoffwechsels unterschiedlich stark exprimiert werden, d. h. ob quantitativ meßbare Unterschiede in der Promotorstärke des pcbAB- und des pcbC-Gens in A. chrysogenum bestehen.

Die Auswahl von Kotransformanten für die Analyse der Genexpression erfolgte anhand der Ergebnisse der "Southern-Hybridisierung". Im quantitativen Testverfahren wurden verschiedene Transformanten untersucht. Die Fermentationsbedingungen und die Kulturdauer von 2,5 Tagen war für alle Kotransformanten wie auch für den Rezipientenstamm gleich. Die Enzymaktivitäten wurden in Protoplasten-Lysaten mit Hilfe eines sensitiven fluorometrischen Testverfahrens gemessen. Für die Bestimmung der $\beta$-D-Galaktosidase-Aktivität wurde als Substrat MUG-Z (4-Methylumbelliferylgalaktosid) verwendet, welches das Enzym in das fluoreszierende Produkt MU (4-Methylumbelliferon) spaltet. Die Messung des umgesetzten Produktes im Proteinextrakt erfolgte nach 1 h Inkubation bei 37 ° C. Die $\beta$-D-Glukuronidase-Aktivität wurde unter Verwendung des Substrates MUG-A (4-Methylumbelliferylglukuronid) bestimmt, welches das Enzym in das fluoreszierende Produkt MU spaltet. Nach 4 h Inkubation bei 37 ° C wurde die im Proteinextrakt umgesetzte Produktmenge gemessen. MU wurde auch als Konzentrations-Standard für die Kalibrierung des Spektralfluorometers eingesetzt. Die anschließende Bestimmung der Gesamtprotein-Konzentration in den Extrakten erfolgte nach Bradford (1976).

Die Ergebnisse des quantitativen Tests zeigt die Tabelle 5.

Tab. 5: Spezifische Enzymaktivitäten in Proteinextrakten verschiedener Kotransformanten von A. chrysogenum. Die spezifische $\beta$-D-Galaktosidase- und $\beta$-D-Glukuronidase-Aktivität wird ausgedrückt in nmol MU x mg Protein$^{-1}$ x h$^{-1}$ (37° C) (Dreifachbestimmung). Die Standard-Abweichung ist angegeben.

| Transkriptions-Fusion | | Spezifische Enzymaktivitäten[a] (nmol MU x mg Protein$^{-1}$ x h$^{-1}$) | |
|---|---|---|---|
| Vektor | Kotransformat | $\beta$-D-Galaktosidase[b] | $\beta$-D-Glukuronidase[c] |
| pcbAB-lacZ | TR1 | 99 (± 15) | |
| (pSRM9.9) | TR2 | 68 (± 18) | |
| | TR3 | 112 (± 9) | |
| | TR4 | 63 (± 11) | |
| | TR5 | 87 (± 8) | |
| | TR6 | 100 (± 11) | |
| | TR7 | 85 (± 8) | |
| | TR8 | 76 (± 9) | |

| Transkriptions-Fusion | | Spezifische Enzymaktivitäten[a] $(nmol\ MU \times mg\ Protein^{-1} \times h^{-1})$ | |
|---|---|---|---|
| Vektor | Kotransformat | $\beta$-D-Galaktosidase[b] | $\beta$-D-Glukuronidase[c] |
| pcbC-lacZ | TI1 | 356 (± 23) | |
| (pSIM9.9) | TI2 | 492 (± 11) | |
| | TI3 | 521 (± 22) | |
| | TI4 | 298 (± 6) | |
| | TI5 | 387 (± 8) | |
| | TI6 | 493 (± 21) | |
| | TI7 | 329 (± 17) | |
| | TI8 | 425 (± 6) | |
| pcbAB-gusA | TI1 | | 26 (± 9) |
| (pSIM9.9) | TI2 | | 0 |
| | TI3 | | 31 (± 11) |
| | TI4 | | 14 (± 5) |
| | TI5 | | 5 (± 3) |
| | TI6 | | 28 (± 3) |
| | TI7 | | 23 (± 6) |
| | TI8 | | 25 (± 8) |
| pcbC-gusA | TR1 | | 148 (± 21) |
| (pSRM9.9) | TR2 | | 130 (± 6) |
| | TR3 | | 115 (± 10) |
| | TR4 | | 132 (± 9) |
| | TR5 | | 80 (± 14) |
| | TR6 | | 128 (± 13) |
| | TR7 | | 113 (± 8) |
| | TR8 | | 102 (± 1) |

a) Die "Hintergrund-Aktivität" an $\beta$-D-Galaktosidase und $\beta$-D-Glukuronidase (Spontanspaltung von MUG-Z bzw. MUG-A in der Negativkontrolle und die endogenen Enzymaktivitäten des Rezipientenstammes A. chrysogenum ATCC14553) sind von den erhaltenen Meßwerten subtrahiert.

b) Die endogenen $\beta$-D-Galaktosidase-Aktivität des Rezipienten betrug 10.2 ( $\pm$ 4). Die Expression des lacZ-Reportergens, die in Form der pcbC-lacZ (pSIM9.9) bzw. pcbAB-lacZ (pSRM9.9) Transkriptions-Fusionen erzielt wurde, wird als spezifische $\beta$-D-Galaktosidase-Aktivität bestimmt.

c) Die $\beta$-D-Glukuronidase-Aktivität betrug 0.06 ( $\pm$ 0.01) nmol MU x mg Protein$^{-1}$ x h$^{-1}$. Die Expression des gus-A-Reportergens in Form der pcbAB-gusA (pSIM9.9) bzw. pcbC-gusA (pSRM9.9) Transkription-Fusionen wurde als spezifische Aktivität der $\beta$-D-Glukuronidase ermittelt.

Die Kotransformanten von A. chrysogenum exprimieren offensichtlich beide Reportergene. Die Expression der Reportergene steht dabei unter der Kontrolle des intergenischen Bereichs. Der Rezipientenstamm zeigt hingegen im quantitativen Test eine geringe endogene $\beta$-D-Glukuronidase-Aktivität und ein bestimmtes Niveau an $\beta$-D-Galaktosidase-Aktivität. Das Ergebnis des quantitativen Tests ist, daß der intergenische Bereich in Richtung des ursprünglichen pcbC-Gens eine wesentlich höhere Expression ergibt als in Richtung des ursprünglichen pcbAB-Gens. Unabhängig von den verwendeten Reportergenen ist erkennbar, daß bei den Kotransformanten die spezifischen $\beta$-D-Galaktosidase-Aktivitäten, die durch den intergenischen Bereich in Richtung des ursprünglichen pcbC-Gens hervorgerufen werden, ca. 5- bis 6mal größer sind als die Enzymaktivitäten, die durch den intergenischen Bereich in Richtung des ursprünglichen pcbAB-Gens hervorgerufen werden. Folglich handelt es sich bei dem intergenischen Bereich um einen bidirektionalen Promotor, der überraschenderweise in Richtung des ursprünglichen pcbC-Gens ca. 5 bis 6mal aktiver ist als in die andere Richtung.

Methode: Quantifizierung der Expression von Reportergenen

Herstellung der Protein-Extrakte

Ein 500 ml Erlenmeyerkolben mit 100 ml CCM wurde mit 5 Tage altem Myzel von A. chrysogenum (-Kotransformanten) angeimpft, für 2,5 Tage kultiviert und protoplastiert. Nach mikroskopischer Kontrolle der Protoplastierung wurden die Protoplasten über Trichter mit Glaswolle vom Myzel getrennt, für 5 min mit 3000 Upm pelletiert, viermal mit je 10 ml Protoplastenpuffer (PP) gewaschen, abzentrifugiert und in 2 ml PP resuspendiert (PP: 10 mM Tris•HCl, 10 mM MgSO$_4$ • 7H$_2$O, 1 M KCl, pH 7,0). Die Protoplastensuspension wurde gleichmäßig auf zwei Eppendorfgefäße (lacZ-Ansatz, gusA-Ansatz) verteilt.

Die Lyse der Protoplasten im gusA-Ansatz erfolgte modifiziert nach Jefferson (1987) und nach Murray et al. (1992). Die Protoplastensuspension des gusA-Ansatzes wurde für 5 min bei 3000 Upm zentrifugiert und das Pellet in 1 ml gusA-Extruktionspuffer (GEP) (50mM NaH$_2$PO$_4$, pH 7.0, 10 mM EDTA, 0.1 % Triton X-100, 0,1 % Natriumlauryl-Sarkosin, 10 mM $\beta$-Merkaptoethanol) gelöst.

Die Lyse der Protoplasten im lacZ-Ansatz erfolgte verändert nach Miller (1972). Nach Zentrifugation der Protoplastensuspension des lacZ-Ansatzes wurde das Pellet in 1 ml lacZ-Puffer (ZP) (25 mM Tris-HCl, pH 7,5, 125 mM NaCl, 2 mM MgCl$_2$, 12 mM $\beta$-Merkaptoethanol) aufgenommen. In beiden Ansätzen wurde die Lyse durch eine Ultraschallbehandlung vervollständigt. Der Aufschluß der Protoplasten erfolgte durch Ultraschall (5 Sekunden bei 50 Watt) und wurde mikroskopisch kontrolliert. Beide Ansätze wurden bis zur Bestimmung der enzymatischen Aktivität auf Eis inkubiert oder aber in flüssigem Stickstoff eingefroren und bei -70° C gelagert. Vor der fluorometrischen Bestimmung wurden die Ansätze für 5 min zentrifugiert und der zu testende Extrakt aus dem Überstand entnommen.

EP 0 618 298 A2

Fluorometrische Bestimmung der gusA-Genexpression

Die fluorometrische Bestimmung der gusA-Genexpression erfolgte modifiziert nach Jefferson (1987). Für jeden Testansatz (Dreifachbestimmung) wurden 250 $\mu$l gusA-Testpuffer (GAP) (2 mM 4-Methylumbelliferyl-A3 -D-Glukuronid (MUG-A) in GEP), 200 $\mu$lGEP und 50 $\mu$l Extrakt aus dem Überstand des gusA-Ansatzes in ein Eppendorfgefäß gegeben. Die Substrat-Zerfallskontrolle (Dreifachbestimmung) enthielt 250 $\mu$l GAP, 200 $\mu$ GEP und 50 $\mu$l A. dest. Zum Zeitpunkt t = 0 und zu anderen gewählten Zeitpunkten (z. B. t = 4h) nach der Inkubation bei 37° C wurde dem Testansatz bzw. der Substrat-Zerfallskontrolle ein 50 $\mu$l-Aliquot entnommen und in 1.95 ml gusA-Stopp-Puffer (GOP) (0.2 M $Na_2CO_3$, pH 10.4) überführt. Das bei der Spaltung des Substrates 4-Methylumbelliferyl-$\beta$-D-Glukuronid (MUG-A) durch die $\beta$-D-Glukuronidase-Aktivität entstandene Produkt 4-Methylumbellliferon (MU) wurde im kalibrierten Spektralfluorometer bestimmt. Die fluorometrische Messung von MU efolgte bei einer Excitations-Wellenlänge von 365 nm und einer Bandbreite von 3 nm; die Emissions-Wellenlänge betrug 455 nm und die Bandbreite 10 nm.

Fluorometrische Bestimmung der lacZ-Genexpression

Die fluorometrische Bestimmung der lacZ-Genexpression wurde verändert nach Miller (1972) durchgeführt. Für jeden Testansatz (Dreifachbestimmung) wurden 250 $\mu$l lacZ-Testpuffer (ZAP) (2mM 4-Methylumbelliferyl-$\beta$-D-Galaktosid (MUG-Z) in ZP), 200 $\mu$l ZP und 50 $\mu$l Extrakt aus dem Überstand des lacZ-Ansatzes in ein Eppendorfgefäß gegeben. Die Substrat-Zerfallskontrolle (Dreifachbestimmung) enthielt 250 $\mu$l ZAP, 200 $\mu$l ZP und 50 $\mu$l A. dest. Zum Zeitpunkt t = 0 und zu anderen gewählten Zeitpunkten (z. B. t = 1 h) nach der Inkubationszeit bei 37° C wurde dem Testansatz bzw. der Substrat-Zerfallskontrolle ein 50 $\mu$l-Aliquot entnommen und die Reaktion durch Zugabe von 50 $\mu$l A. des. und 50 $\mu$l 25 % Trichloressigsäure (TCA) unter Abkühlung auf Eis abgestoppt. Nach Zentrifugation für 2 min mit 12000 Upm wurden 50 $\mu$l des Überstandes zu 1.95 ml lacZ-Stopp-Puffer (ZOP) (133 mM Glycin, 83 mM $Na_2CO_3$, pH 10.7) gegeben. Das bei der Spaltung von 4-Methylumbelliferyl-$\beta$-D-Galaktosid (MUG-Z) durch die $\beta$-D-Galaktosidase-Aktivität entstandene Produkt 4-Methylumbelliferon (MU) wurde im kalibrierten Spektralfluorometer bestimmt.

Bestimmung der Gesamtprotein-Konzentration

Die Bestimmung der im gusA- bzw. lacZ-Ansatz enthaltenen Gesamtprotein-Konzentration erfolgte nach Bradford (1976). Jedem Testansatz (Dreifachbestimmung) wurde ein 5 $\mu$l-Aliquot entnommen und in 95 $\mu$l A. dest. überführt. Nach Zugabe von 1 ml fünffach verdünnter Bradford-Stammlösung (0.325 % Serva Blau G, 0.675 % 85 %ige Phosphorsäure) wurde sofort die Extinktion bei einer Wellenlänge von 595 nm im kalibrierten Photometer (Gilford) bestimmt.

Literatur

Bevan MD, Gibson TJ, Hong GF (1983) Buffer gradient gels and 35S label as an aid to rapid DNA sequence determination. Proc Natl Acad Sci USA 80: 3963-3965
Bradford MM (1976) A rapid and sensitive method for the quantitation of microgram quantities of protein utilizing the principle of protein-dye binding. Anal Biochem 72: 248-254
Gutierrrez S, Diez B, Montenegro E, Martin JF (1991) Characterization of the Cephalosporium acremonium pcbAB gene encoding alpha-aminoadipyl-cysteinylvaline synthetase, a lage multidomain peptide synthetase: linkage to the pcbC gene as a cluster of early cephalosporin biosynthetic genes and evidence of multiple functional domains. J Bacteriol 173: 2354-2365.
Herrera-Estrella L, Teeri TH, Simpson J (1988) Use of reporter genes to study gene expression in plant cells. In: Plant molecular biology manual-B1-Expression of genes in plants Gelvin SB, Schilperoort RA, Verma DS (eds), Dordrecht, Kluwer Academic Publishers, pp 1-22.
Jefferson RA, Burgess SM, Hirsch D (1986) $\beta$-Glucuronidase from Escherichia coli as a gene-fusion marker. Proc Natl Acad Sci USA 83: 8447-8451
Kück U, Walz M, Mohr G, Mracek M (1989) The 5'-sequence of isopenicillin N-synthetase gene (pcbC) from Cephalosporium acremonium directs the expression of the prokaryotic hygromycin B phosphotransferase gene (hph) in Aspergillus niger. Appl Microbiol Biotechnol 31: 358-365
MacGregor GR (1987) Histochemical staining of clonal mammalian cell lines expressing E. coli $\beta$-galactosidase indicates heterogeneous expression of the bacterial gene. Somat Cell Mol Genet 13: 253-265
Miller JH (1972) Experiments in molecular genetics. Cold Spring Harbor Laboratory Press. Cold Spring Harbor, New York

Murray FR, Latch GCM DB (1992) Surrogate transformation of perennial ryegrass, Lolium perenne, using genetically modified Acremonium endophyte. Mol Gen Genet 233: 1-9

Richard G, Bailey JA, Keon PR, Hargreaves JA (1992) Development of a GUS reporter gene system for the maize pathogen Ustilago maydis. Phys Mol Plant Pat 40: 383-393

Sanger F, Nicklen S, Coulsen AR (1977) DNA sequencing with chain-terminating inhibitors. Proc Natr Acad Sci USA 74:5463-5467

Walz M (1992) Molekulare Analysen zur Expression von $\beta$-Lactam-Genen bei Acremonium chrysogenum. Bibliotheca Mycologica, J Cramer, Gebrüder Bornträger Verlagsbuchhandlung, Berlin, Stuttgart, Band 147

## Sequenzprotokoll

Allgemeine Angaben:

Anmelder:　　Hoechst AG

　　　　　　65926 Frankfurt am Main

　　　　　　Telefon: (069) 305-6031

　　　　　　Telefax: (069) 35 71 75

Bezeichnung der Erfindung:　　Bidirektionaler Promoter des pcbAB- und pcb-C-Gens von Acremonium chrysogenum und seine Verwendung

Anzahl der Sequenzen: 7

Computerlesbare Fassung:

Datenträger: 3,5'' HD Diskette

Computer: 386 SX

Betriebssystem: MS-DOS

Software: ASCII

Angaben zu SEQ ID NO: 1

Sequenzkennzeichen:

Länge: 18 Basen

Art: Nucleinsäure

Strangform: Einzelstrang

Topologie: linear

Ursprüngliche Herkunft:

Organismus: Acremonium chrysogenum

Unmittelbare Herkunft: synthetisch

Merkmal: intergenischer Bereich

Sequenzbeschreibung: SEQ ID NO: 1

AGGCCAATGC ATGCATCC

Angaben zu SEQ ID NO: 2

Sequenzkennzeichen:

Länge: 18 Basen
Art: Nucleinsäure
Strangform: Einzelstrang
Topologie: linear

Ursprüngliche Herkunft:

Organismus: Acremonium chrysogenum

Unmittelbare Herkunft: synthetisch

Merkmal: intergenischer Bereich

Sequenzbeschreibung: SEQ ID No: 2

AACAGTCTAT CGAAGCTC

SEQ ID NO.: 4

```
          10        20        30        40        50        60
CACGGACCGG ATCCAGCAGT CAGGGGCTGC GGCTGCGAAG CCGTTGCGGT GGATGGCACC

          70        80        90       100       110       120
TTTTGGGATA TCAGAGCTTC CATTGCGCCT GATGTGGTAA TGTTAGAGAT AGCTCCCAAC

         130       140       150       160       170       180
TGCGCTTTCC AGAGGCTGTG AAGACATACC TGTGGTATGA CGAGGCGTCG TCGGCACCTG

         190       200       210       220       230       240
TAAGGATAGT CATGTTGATA TCAAAGGTGT ATGATAGACT GGCAGTCCAC TACTGACTGA

         250       260       270       280       290       300
GTGTGACCGA GTCCAAGGAG AAATATTTCC GCCAGTTCTT TTTCGCACTC TACGGCCGAT

         310       320       330       340       350       360
ATTTCCCAAC ACGTCGCTAG ATCTCCCAGC ACGAACGTCT TATACTCCTG GGGAATCCCG

         370       380       390       400       410       420
GACCTTTGAT ACAGGCCAAT GCATGCATCC AGCTGGCCAC CCACCCAGAC CAGTGAACTC

         430       440       450       460       470       480
CAAAGCCGGC ACCGAACCCA CCAGGACCGC CTTTGTCGCC ATGGATACCC TTTCTCTCCT

         490       500       510       520       530       540
CGGCCAAGCG CGATGGCCCG CGTCTTGCAG AAAGCTGGGC AGGCTTCAGA AGTGGGCCAC

         550       560       570       580       590       600
GCTGCAGGTC GGTCCCTCGT CTGCGAGTTT AAAGTGCAAC GTGAACCAAA GCCACCCCGT

         610       620       630       640       650       660
ATGTGTGGTT CCATTGGGTT GAGAAACTGG ATTGTACGGG GTACCTCTAC CCCGAGTTCC

         670       680       690       700       710       720
TCGTCGATCG GGTACATGAT ACATTGTACA CCGTAGTAGC AGTGTGTACG GCCCATCGCC

         730       740       750       760       770       780
GTGGTGGCCC GCAGCAGCGC GAGATTCCGA CGGACGCCGT CGACTACCGG TGAGCCGCTC

         790       800       810       820       830       840
GACGGGGCGT CGAGTTGCCG GGCCAATCCC TGAGCTTCGA TAGACTGTTC CGGGCCTCAT

         850       860       870       880       890       900
GGGTGGCGGC GTCTACATGC ACATGCATGT AACGGCGTTC CTCATCGCTT GGCCCCGCCA

         910       920       930       940       950       960
TGCAGTCTTC AGGGACCAAA CTCCATCGCC GCTGCTGGAC CGTATGTAAC CCCCCTCGGC

         970       980       990      1000      1010      1020
AGTGTCACCC GCAGGAGCCG GATAATCGAG ACCTTGGTCA GGCCATAAAG GCGCGTCGTG

        1030      1040      1050      1060      1070      1080
GGGAAGCTCA TATCGTATAG CAACGGGAGA CACGAGGTAG GTACTCAAGT ACACATACAC

        1090      1100      1110      1120      1130      1140
ACACCCAGCC GCCCGTATAA ACAGCTTCAA GAGGGGCGAA TACTTGAATA TCCCTTTGGT

        1150      1160      1170      1180      1190      1200
CGCTCTTCTG ATTTTCGAGG CTTCTCCTTC CGCCATCGTC ACTCACGCAT ATCTCGTCTT

        1210      1220      1230      1240      1250      1260
TCACATCTTA CACCAGGCAG GACAAACCGT CACCATG
```

16

Angaben zu SEQ ID NO: 3

Sequenzkennzeichen:

Länge: 21 Basen
Art: Nucleinsäure
Strangform: Einzelstrang
Topologie: linear

Ursprüngliche Herkunft:

Organismus: Acremonium chrysogenum

Unmittelbare Herkunft: synthetisch

Merkmal: intergenischer Bereich

Sequenzbeschreibung: SEQ ID NO: 3

AAAGGGATAT  TCAAGTATTC  G

Angaben zu SEQ ID NO: 4

Sequenzkennzeichen:

Länge: 1237 Basen
Art: Nucleinsäure
Strangform: Einzelstrang
Topologie: linear

Ursprüngliche Herkunft:

Organismus: Acremonium chrysogenum

Unmittelbare Herkunft: genomisch

Merkmal: intergenischer Bereich

Sequenzbeschreibung: SEQ ID NO: 4

Angaben zu SEQ ID NO: 5

Sequenzkennzeichen:

Länge: 76
Art: Nucleinsäure
Strangform: Einzelstrang
Topology: linear

Merkmal:    DNA-Sequenz der Übergangsbereiche in den Vektoren pSI8.8

Sequenzbeschreibung: SEQ ID NO: 5

TAACATAAGG    GACTGACCAC    CCGGG.................... CCCGGGATCG
AAAGAGCCTG    CTAAAGCAAA    AAAGAAGTCA    CCATGTCGTT  T

Angaben zu SEQ ID NO: 6

Sequenzkennzeichen:

Länge: 100
Art: Nucleinsäure
Strangform: Einzelstrang
Topology: linear

Merkmal:    DNA-Sequenz der Übergangsbereiche in den Vektoren pSI8.8

Sequenzbeschreibung: SEQ ID NO: 6

TAACATAAGG    GACTGACCAC    CCCAGCAGTC    AGGGGCTGC...

GACAAACCGT    CACGGGATCG    AAAGAGCCTG    CTAAAGCAAA

AAAGAAGTCA    CCATGTCGTT    T

Angaben zu SEQ ID NO: 7

Sequenzkennzeichen:

Länge: 69

Art: Nucleinsäure

Strangform: Einzelstrang

Topology: linear

Merkmal:    DNA-Sequenz der Übergangsbereiche in den Vektoren pSI8.8

Sequenzbeschreibung ID NO: 7

TAACATAAGG    GACTGACCAC    CCTGACGGTT    TGTC.............

GCAGCCCCTG    ACTGCTGGGG    ATCGAAAGAG    CCTGCTAAAG

CAAAAAAGAA    GTCACCATGT    CGTTT

**Patentansprüche**

1. DNA-Segment gemäß Tab. 2 oder Teile davon, die eine bidirektionale Promotoraktivität besitzen.

2. DNA-Segment, das mit einer DNA gemäß Tab. 2 unter stringenten Bedingungen hybridisiert und eine bidirektionale Promotoraktivität besitzt.

3. Rekombinierte DNA enthaltend ein DNA-Segment nach Anspruch 1 oder 2 und ein oder zwei DNA-Segmente, die für ein Protein kodieren, wobei die proteinkodierenden DNA-Segmente mit dem promotoraktiven DNA-Segment funktionell verbunden sind.

4. DNA nach Anspruch 3, wobei die proteinkodierenden DNA-Segmente für gusA und/oder lacZ kodieren.

5. Vektor enthaltend eine DNA nach Anspruch 1 oder 2.

6. Expressionsvektor enthaltend eine DNA nach Anspruch 3 oder 4.

7. Vektor pSRM9.9 oder pSIM9.9 gemäß Fig. 2b.

8. Wirtszelle, insbesondere Acromonium chrysogenum oder Aspergillus niger, enthaltend einen Vektor nach einem der Ansprüch 5 - 7.

9. Verfahren zur Herstellung eines gewünschten Proteins, dadurch gekennzeichnet, daß eine Wirtszelle nach Anspruch 8 kultiviert und das gewünschte Protein isoliert wird.

10. Verwendung einer DNA nach einem der Ansprüche 1 - 3 zur Regulation der Transkription von Fremd-DNA in Acremonium chrysogenum.

11. Verwendung einer DNA nach einem der Ansprüche 1 - 4 zur quantitativen Bestimmung der Genexpression von Reportergenen in Acremonium chrysogenum.

Fig. 1

EP 0 618 298 A2

Fig. 2